# EUROPÄISCHE PATENTANMELDUNG

(11) **EP 1 376 187 A1**
(43) Veröffentlichungstag der Anmeldung: **02.01.2004**
(21) Anmeldenummer: 03011489.6
(22) Anmeldetag: 21.05.2003
(51) Int. Cl.: G02B 21/00

(54) **Sprachsteuerung für Operationsmikroskope**

(30) Priorität: 14.06.2002 DE 10226539
(71) Anmelder: Leica Microsystems (Schweiz) AG, 9435 Heerbrugg (CH)
(72) Erfinder: Sander, Ulrich, Dr., 9445 Rebstein (CH); Lippe, Michael, Winter Haven, FL (US); Savanayana, Ohm, 9434 Au (CH); Engl, Jürgen, 89233 Neu-Ulm (DE); Tedde, Andreas, 9452 Hinterforst (CH)
(74) Vertreter: Reichert, Werner F., Dr.

(57) **Zusammenfassung**

Die Erfindung betrifft eine Steuerungsvorrichtung, insbesondere für Operationsmikroskope (8), mit einer Sprachsteuerung (2), bei welcher ein Teil der Funktionen mittels Hand- (4) und/oder Fuß- (5) und/oder Augensteuerung (7) und ein anderer Teil der Funktionen mittels der Sprachsteuerung (2) ausführbar sind.

## Beschreibung

Die Erfindung betrifft eine Sprachsteuerung z. B. für Operationsmikroskope. Sprachsteuerungssysteme halten Einzug bei sämtlichen Steuerungen von Maschinen, Vorrichtungen und Computern und sind insbesondere da von großem Nutzen, wo die Hände oder Finger für ein konzentriertes Arbeiten gebraucht werden. Im Bereich der Medizin und speziell im Bereich der Operationsmikroskope ist es zudem wünschenswert, einerseits das konzentrierte und genaue Arbeiten der Hände nicht zu unterbrechen, andererseits aber auch die höchsten Sterilitätserfordernisse nicht unnötig weit ausdehnen zu müssen.

Insbesondere letzterem Gedanken trägt das SIOS (Siemens Integriertes OP-System, A.Schafmayer, D. Lehmann-Beckow, M. Holzner "The Process-Optimized Operating Room" in electromedica 68/2000 no.2) Rechnung, weil hiermit mittels einer zur sterilen Fernbedienung optionalen Sprachsteuerung die Funktionen im und rund um den OP-Bereich per Sprache angesteuert werden können, also ohne dass eine Bedienperson - sei es im sterilen oder im unsterilen Bereich - die Handlung selber vornehmen müsste. Allerdings umfasst dieses bekannte Sprachsteuerungssystem das Operationsmikroskop selbst nicht.

Um die Hände des Chirurgen in ihrer Arbeit nicht zu unterbrechen, sind auch Lösungen mit Fußpedalen realisiert worden. Diese hatten jedoch den Nachteil, den für das genaue Arbeiten erforderlichen ruhigen Stand des Chirurgen zu beeinträchtigen. Demzufolge bestand ein gesteigertes Bedürfnis, Sprachsteuerungen für die Steuerung von Operationsmikroskopen zu realisieren.

Um zu vermeiden, dass gerade in hochsensiblen Bereichen falsche Befehle zur Ausführung gelangen, wurden sprecherabhängige Systeme, wie z. B. "Katalavox" von Kempf, Sunnyvale, California (US), entwickelt, welche auch in lauten Umgebungen verlässlich funktionieren.

Alle bekannten Sprachsteuerungen erstrecken sich auch auf sogenannte kontinuierliche Funktionen, sei es auf das Anheben oder das Senken eines OP-Tisches, sei es auf das Dimmen einer Beleuchtung, sei es auf das Ausrichten von Zusatzgeräten oder auf das Zoomen oder Fokussieren eines Operationsmikroskops. Bei diesen kontinuierlichen Funktionen erwies es sich jedoch als nachteilig, durch Wiederholung des gesprochenen Befehls den gewünschten Anfahrpunkt schrittweise und mit umständlich vielen Befehlen zu erreichen. Zudem bestand die Gefahr, dass mit einem ganzen Schritt zu leicht über den erwünschten Punkt hinausgefahren wird. Als wünschenswert erwies es sich, den gesuchten Anfahrpunkt aus weiter Entfernung rasch anzufahren, um sich dann möglichst fein und langsam dem Punkt nähern zu können. Im Unterschied hierzu stehen sogenannte "Ein-Aus"-Funktionen, welche sehr gut durch die Sprachsteuerung bedient werden können.

Aufgabe der Erfindung ist es somit, auf die Vorteile einer Sprachsteuerung nicht zu verzichten, dabei jedoch zusätzlich das Ansteuern der kontinuierlichen Funktionen zu verbessern.

Gelöst wird diese Aufgabe durch einen Rückgriff auf eine - bevorzugt optionale - manuelle Steuerung und/oder Fuß- und/oder Augensteuerung (Eye-Control), bei welcher aber allerdings erfindungsgemäß das Aufrufen der übergeordneten Funktion mittels Sprachsteuerung geschieht. Letzteres soll aber nicht im Sinne vorliegender Erfindung als zwingende Ausgestaltung angesehen werden.

Erfindungsgemäß sollen die "Ein-Aus"-Funktionen mit der Sprachsteuerung bedient werden können, die kontinuierlichen Funktionen hingegen mittels der Sprachsteuerung aufrufbar sein, aber die Funktion selbst per Tastendruck am Handgriff oder Fußpedale oder dergleichen (z. B. auch Eye-Control) auszuführen sein.

Eine bevorzugte Ausgestaltung der Erfindung sieht vor, dass die Sprachsteuerung über ein von ihr entkoppeltes Sprachbedienmodul verfügt. Die Entkoppelung des Sprachbedienmoduls von der Sprachsteuerung bzw. von dem Operationsmikroskop bringt nämlich den Vorteil, dass nur das Sprachbedienmodul oder auch umgekehrt nur das Steuergerät bzw. Operationsmikroskop sterilisiert werden muss. So wäre der sterile Chirurg z. B. in der Lage, das Operationsmikroskop, welches noch außerhalb des sterilen Bereichs von den OP-Schwestern vorbereitet wird, vorab mittels des Sprachbedienmoduls zu aktivieren und vorab die gewünschten Einstellungen vorzunehmen.

Sobald das Sprachbedienmodul aktiv ist, sind sämtliche Funktionen des Operationsmikroskops aufrufbar und erfindungsgemäß sind dann bestimmte Funktionen, wie z. B. die Zoom- oder die Fokus-Funktion, über die Tastatur des Handgriffs oder die Fußsteuerung oder mit dem Eye-Control bedienbar. Gemäß einer bevorzugten Ausgestaltung der Erfindung ergibt sich für den Chirurgen folgender Bedien-Ablauf: Er schaltet per Sprachbefehl, z. B. "Sprachsteuerung ein", das Sprachbedienmodul ein. Daraufhin ruft er mit z. B. "Zoomen" die Zoom-Funktion auf und ist dann in der Lage, mit der Hand-, Fuß- oder Augensteuerung, jedenfalls - nach einer bevorzugten Ausgestaltung optional - **nicht** mit der Sprachsteuerung das Zoom zu bedienen.

Andererseits ist in einer weiterhin bevorzugten Ausgestaltung der Erfindung auch ein "Stand-by"-Modus der Sprachsteuerung vorgesehen, in welchem **alle** Funktionen des Operationsmikroskops - auch im Falle eines Ausfalls der Sprachsteuerung - herkömmlich über die Hand- und/oder Fuß- und/oder Augensteuerung bedienbar sind.

Eine andere Ausgestaltung der Erfindung sieht vor, dass mit einem bestimmten Sprachbefehl die Sprachsteuerung in einen Modus geschaltet wird, in welchem die kontinuierlichen Funktionen so lange ausgeübt werden, solange ein bestimmter Laut (Vokal) - vielleicht zusätzlich auch sprecheridentifiziert - vorgetragen wird. Sobald mit dem Aufsagen dieses Lautes innegehalten wird, wird die Ausübung der angewählten Funktion sofort gestoppt. Verschiedene Laute oder verschiedene Tonlagen entsprechen verschiedenen Ausübungsgeschwindigkeiten der Funktion. Auch ist es möglich, dass das Sprachbedienmodul über eine elektronische Datenleitung, insbesondere über einen CAN-Bus und/oder über Lichtwellenleiter und/oder eine Sende-Empfangsverbindung mit vorzugsweise Funkund/oder Lichtwellen, insbesondere im infraroten Bereich, mit dem Steuergerät verbunden ist.

Weitere Ausbildungen der Erfindung sind in den Patentansprüchen angegeben.

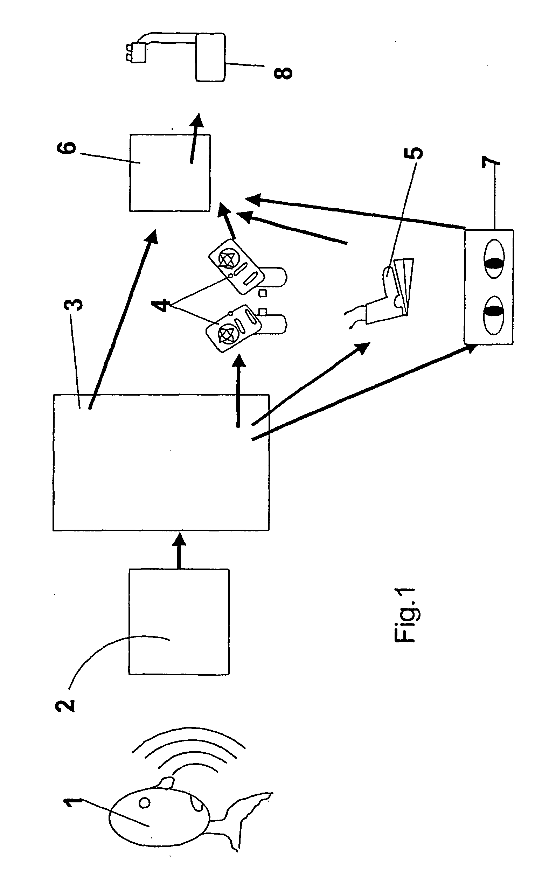

Anhand einer Figur wird die Erfindung symbolisch und beispielhaft näher erläutert. Es zeigt dabei
- Fig.1:: eine schematische Darstellung der Anordnung der Sprachsteuerung, des Bedien-Menüs, der Hand-, der Fuß-, der Augensteuerung (Eye-Control), des Steuergeräts und des Operationsmikroskops.

In Fig.1 ist ersichtlich, dass eine Bedienperson 1 über ein Sprachbedienmodul 2 ein Bedien-Menü 3 aufrufen kann und hierbei unterschieden werden muss zwischen Funktionen, bei welchen das Steuergerät 6 direkt aktiviert wird und anderen, bei welchen die Aktivierung des Steuergeräts 6 über die Handgriffe 4 und/oder die Fußsteuerung 5 und/oder das Augensteuerungs-Bedienmodul (Eye-Control) 7 erfolgt. Das Steuergerät 6 ist mit dem Operationsmikroskop 8 verbunden.

### Bezugszeichenliste

- 1 -: Bedienperson
- 2 -: Sprachbedienmodul
- 3 -: Bedien-Menü
- 4 -: manuelles Bedienmodul (Handsteuerung)
- 5 -: Fußbedienmodul (Fußsteuerung)
- 6 -: Steuergerät
- 7 -: Augensteuerungs-Bedienmodul (Eye-Control)
- 8 -: Operationsmikroskop

## Patentansprüche

1. Steuerungsvorrichtung für ein Gerät, insbesondere für ein Operationsmikroskop (8), mit einem Sprachbedienmodul (2), **dadurch gekennzeichnet, dass** dem Sprachbedienmodul (2) wenigstens ein weiteres Bedienmodul (4, 5, 7) zugeordnet ist, sodass ein Teil der steuerbaren Funktionen des Geräts ausschließlich über das Sprachbedienmodul (2) anwählbar ist und ein anderer Teil der steuerbaren Funktionen entweder auch über das Sprachbedienmodul (2) oder ausschließlich über mindestens eines der anderen Bedienmodule (4, 5, 7) anwählbar ist.

2. Steuerungsvorrichtung nach Anspruch 1, **dadurch gekennzeichnet, dass** sie ein Steuergerät (6) für kontinuierliche Funktionen umfasst, das mit einem manuellen Bedienmodul (4) und/oder mit einem Fußbedienmodul (5) und/ oder mit einem Augensteuerungs-Bedienmodul (7) verbunden ist.

3. Steuerungsvorrichtung nach einem der vorhergehenden Ansprüche, **dadurch gekennzeichnet, dass** das Sprachbedienmodul (2) eine Stand-by-Funktion hat, in welcher alle Funktionen dem Hand- (4) und/oder dem Fuß- (5) und/oder dem Augensteuerungs-Bedienmodul (7) zugeordnet sind.

4. Steuerungsvorrichtung nach einem der vorhergehenden Ansprüche, **dadurch gekennzeichnet, dass** das Sprachbedienmodul (2) eine aktive Bedienmodus-Funktion aufweist, in der nur bestimmte Funktionen dem Hand- (4) und/oder dem Fuß- (5) und/oder dem Augensteuerungs-Bedienmodul (7) zugeordnet sind.

5. Steuerungsvorrichtung nach einem der vorhergehenden Ansprüche, **dadurch gekennzeichnet, dass** das Sprachbedienmodul (2) über eine elektronische Datenleitung verbunden ist und dass das Steuergerät (6) eine automatische Erkennvorrichtung umfasst, welche im Betriebs-zustand automatisch erkennt, ob das Sprachbedienmodul (2) aktiv ist oder nicht.

6. Steuerungsvorrichtung nach einem der vorhergehenden Ansprüche, **dadurch gekennzeichnet, dass** das Sprachbedienmodul (2) sowohl direkt, als auch über das Steuergerät (6) ein- und ausschaltbar ist.

7. Steuerungsvorrichtung nach einem der vorhergehenden Ansprüche, **dadurch gekennzeichnet, dass** das Sprachbedienmodul (2) von dem Steuergerät (6) entkoppelbar ist.

8. Steuerungsvorrichtung nach einem der vorhergehenden Ansprüche, **dadurch gekennzeichnet, dass** ein Display vorgesehen ist, das im Betriebszustand ein Bedien-Menü (3) darstellt, das einen Menüpunkt "Sprachsteuerung" umfasst.

9. Steuerungsvorrichtung nach einem der vorhergehenden Ansprüche, **dadurch gekennzeichnet, dass** sie ein Programm umfasst, bei dem kontinuierliche Funktionen des Geräts über das Sprachsteuerungsmodul (2) und/oder mindestens ein weiteres Bedienmodul (4, 5, 7) anwählbar sind, wobei zusätzlich zu einem bestimmten vorgegebenen Ausführungsbefehl ein Bedienmodus anwählbar ist, in dem die kontinuierliche Funktion so lange ausübbar ist, solange vom Anwender ein bestimmter Laut bzw. Ton an das Sprachsteuerungsmodul (2) vorgetragen wird.

10. Steuerungsvorrichtung nach Anspruch 9, **dadurch gekennzeichnet, dass** verschiedene Laute und/oder verschiedene Tonlagen des vorgetragenen Lautes verschiedene Geschwindigkeiten und/oder Beschleunigungsvorgänge und/oder Abläufe der Funktionsausübung steuern bzw. auslösen.

11. Steuerungsvorrichtung nach einem der vorhergehenden Ansprüche, **dadurch gekennzeichnet, dass** das Sprachsteuerungsmodul (2) ein Mikrofon zur Aufnahme akustischer Laute umfasst, das im Sterilbereich des Geräts bzw. Operationsmikroskops (8) angeordnet ist.

12. Steuerungsvorrichtung nach einem der vorhergehenden Ansprüche 1 bis 10, **dadurch gekennzeichnet, dass** das Sprachsteuerungsmodul (2) ein Mikrofon zur Aufnahme akustischer Laute umfasst, das im Unsteril-Bereich des Geräts bzw. Operationsmikroskops (8) angeordnet ist.
